(19) Europäisches Patentamt
European Patent Office
Office européen des brevets

(11) Publication number: **0 428 272 A2**

(12) **EUROPEAN PATENT APPLICATION**

(21) Application number: **90311388.4**

(51) Int. Cl.[5]: **A61K 31/55**

(22) Date of filing: **17.10.90**

(30) Priority: **17.10.89 US 422992**

(43) Date of publication of application:
**22.05.91 Bulletin 91/21**

(84) Designated Contracting States:
**AT BE CH DE ES FR GB IT LI NL SE**

(71) Applicant: **Ellinwood, Everett Hews**
**3519, Tonbridge Way**
**Durham, North Carolina 27707(US)**

Applicant: **Gupta, Samir Kumar**
**3121, S. LaSalle Street, Apt., 44-E**
**Durham, North Carolina 27705(US)**

(72) Inventor: **Ellinwood, Everett Hews**
**3519, Tonbridge Way**
**Durham, North Carolina 27707(US)**
Inventor: **Gupta, Samir Kumar**
**3121, S. LaSalle Street, Apt., 44-E**
**Durham, North Carolina 27705(US)**

(74) Representative: **Pidgeon, Robert John et al**
**Appleyard Lees & Co. 15 Clare Road**
**Halifax West Yorkshire HX1 2HY(GB)**

(54) **Trifluorobenzodiazepine compounds for use in therapy by intraoral administration.**

(57) The invention relates to trifluorobenzodiazepine compounds for human intraoral administration. Intraoral administration has been found to maximise the $BZ_1$ effects and minimise the $BZ_2$ effects on the human central nervous system in order to maximise the anti-anxiety, anticonvulsant and hypnotic effects and minimise the ataxic and incoordination effects of such compounds.

EP 0 428 272 A2

# TRIFLUOROBENZODIAZEPINE COMPOUNDS FOR USE IN THERAPY BY INTRAORAL ADMINISTRATION

This invention relates to a novel method of administering certain benzodiazepines which surprisingly results in a maximization of the effect on $BZ_1$ receptors and minimization of the effect on $BZ_2$ receptors of the human central nervous system so as to maximize the antianxiety, anticonvulsant, and/or hypnotic effects and to minimize the ataxic and incoordination effects of the drug thereon.

The most pertinent prior art reference known to applicants is U.S. Patent No. 4,229,447 to Porter which discloses a method of administering certain benzodiazepines sublingually and buccally. Porter specifically mentions the sublingual or buccal administration of diazepam, lorazepam, oxazepam, temazepam and chlorodiazepoxide and describes two generic structures of benzodiazepines that may be administered sublingually or buccally. The compound shown below is contemplated by the generic structures in Porter. All of the benzodiazepines disclosed and the generic structure described in Porter are $BZ_1$-$BZ_2$ receptor non-specific since they lack the trifluoro group in the N position of the "B" ring which confers $BZ_1$ specificity.

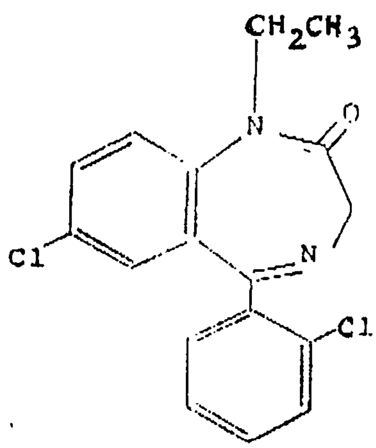

Porter's method is based on the rapid buccal or sublingual absorption of selected benzodiazepines to attain effective plasma concentration more rapidly than oral administration. In contrast, while parenteral administration provides a rapid rise of blood levels of the benzodiazepines, parenteral administration is frequently accompanied by pain and irritation at the injection site and may require sterilization of the preparatives and the hypodermic syringes.

Porter points out that the intraoral, i.e. buccal or sublingual administration, of lipid soluble benzodiazepines results in therapeutic levels resembling parenteral administration without some of the problems associated therewith. Porter's administration technique for benzodiazepines in general builds on a long established knowledge in pharmacology that drugs absorbed in the intraoral route give rise to more rapid absorption than when swallowed into the stomach. What is not recognized by Porter, however, are concerns with first-pass metabolism which can be avoided either with the sublingual or parenteral route of drug administration of certain benzodiazepines.

Porter does not recognize that first-pass metabolism designates the drug absorption directly into the portal blood supply leading to the liver and that the liver in turn rapidly absorbs and metabolizes the drug with its first-pass high concentration through the liver blood supply. Thus, large amounts of the drug may never be seen by the systemic circulation or drug effect site. Porter further does not recognize that the more rapid metabolism via the first-pass metabolism route can lead to accelerated dealkylation with formation of high plasma concentrations of an unwanted metabolite. Thus, applicants' concern with avoiding the degradation of the parent compound and its desired positive effect and the metabolism thereof to an undesired metabolite is neither recognized nor addressed by Porter, which only addresses the ability of the oral mucous membranes to absorb certain benzodiazepines fast and achieve high plasma levels thereof quickly.

The specific drug for which this phenomenon was demonstrated by Porter and lorazepam which has a simple metabolism that results in it not being metabolized to active compounds. Also, and very significantly, the issue of human nervous system receptor specificity and activation for $BZ_1$ and $BZ_2$ type receptors is not recognized by Porter either generally or with reference specifically to trifluorobenzodiazepines.

U.S. Patent No. 3,694,552 to Hester discloses that 3-(5-phenyl-3H-1,4-benzodiazepine-2-yl) carbazic acid alkyl esters, which are useful as sedatives, hypnotics, tranquilizers, muscle relaxants and anticonvulsants, can be administered sublingually. Subsequently issued U.S. Patent No. 4,444,781 to Hester specifically teaches that 8-chloro-1-methanol-6-(o-chlorophenyl)-4H-s-triasolo[4,3-a][1,4]-benzodiazepine therapeutic compounds, which are useful as soporifics, can be suitably prepared for sublingual use.

Also, U.S. Patent No. 4,009,271 to vonBebenburg et al. discloses that 6-aza-3H-1,4-benzodiazepines and 6-aza-1,2-dihydro-3H-1,4-benzodiazepines (which have pharmacodynamic properties including psychosedative and anxiolytic properties as well as antiphlogistic properties) can be administered enterally, parenterally, orally or perlingually.

It is well known by those practiced in the art that special distribution of enzymatic activity within the liver leads to a metabolic zonation for metabolisms of drugs. This zonation is noted in peripheral midzonal and pericentral regions of the liver. Thus, the relative distribution of 2 or more enzymes with respect to substrate entry point and the relative magnitudes of the enzymatic parameters will have a large impact on the metabolic pathway emphasized.

When a drug is swallowed, the stomach and small intestine absorb it with subsequent flow to the portal vein entry to the liver. Thus differential metabolic zonation is possible if the drug is distributed to the liver by the portal vein rather than by the hepatic artery from the general circulation.

Even though this general background information is known to those practiced in the art, the specific findings that trifluorobenzodiazepine N-desalkylation is reduced by sublingual/buccal administration was not known until applicants' unexpected discovery with quazepam and halazepam.

In accordance with the present invention, applicants provide a novel method for maximizing the effect of selected trifluorobenzodiazepines including 7-chloro-1- (2,2,2-trifluorethyl)-5-(o-fluorophenyl)-1,3-dihydro-2H-1,4-benzodiazepine-2-thione (quazepam) and 7-chloro-1,3 dihydro-5-phenyl-1-1- (2,2,2-trifluoroethyl)-2H-1,4-benzodiazepine-2-one (halazepam) on benzodiazepine Type I ($BZ_1$) receptors and minimizing the unwanted potent effect of certain metabolites on benzodiazepine Type II ($BZ_2$) receptors of the human central nervous system so as to maximize the antianxiety and anticonvulsant and/or hypnotic effect sand minimize the ataxic and incoordination effects thereon, comprising selecting a suitable lipid soluble and $BZ_1$ specific trifluorobenzodiazepine, placing the trifluorobenzodiazepine in a suitable intraoral formulation, and intraorally administering a therapeutically effective amount of said intraoral formulation so as to bypass the first pass metabolism of said selected trifluorobenzodiazepine in the liver. The selected trifluorobenzodiazepines with $BZ_1$ specificity are represented by the following structural formula and include:

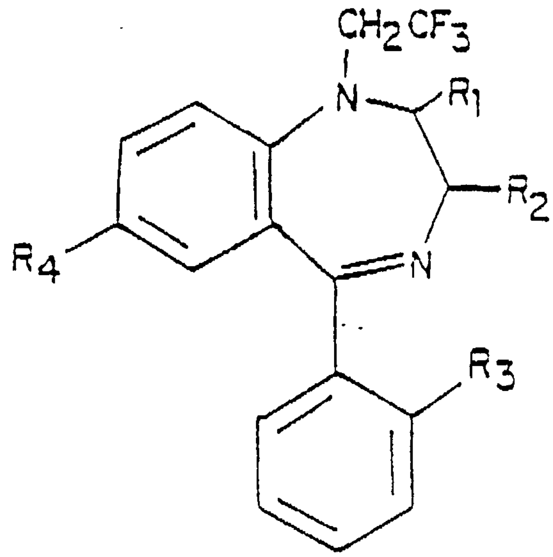

| COMPOUND | $R_1$ | $R_2$ | $R_3$ | $R_4$ |
|---|---|---|---|---|
| 1. HALAZEPAM | =O | H,H | H | CL |
| 2. 3-OH-HALAZEPAM | =O | OH,H | H | CL |
| 3. QUAZEPAM (Q) | =S | H,H | F | CL |
| 4. 2-OXO-Q | =O | H,H | F | CL |
| 5. 2-OXO-3-OH-Q | =O | OH,H | F | CL |
| 6. SCH 15698 | H,H | H,H | F | CL |
| 7. SCH 15893 | H,H | H,H | CL | CL |
| 8. SCH 18449 | H,H | H,H | F | BR |
| 9. 3-OH-Q | =S | OH,H | F | CL |

1. 7-chloro-1-(2,2, 2–trifluoroethyl)-5-phenyl-1,3-dihydro-2H-1,4-benzodiazepin-2-one.
2. 7-chloro-1-(2,2, 2–trifluoroethyl)-5-phenyl-1,3-dihydro-3-hydroxy-2H-1,3-benzodiazepin-2-one.
3. 7-chloro-1-(2,2, 2–trifluoroethyl)-5-(2-fluorophenyl)-1,3-dihydro-2H-1,4-benzodiazepin-2-thione.
4. 7-chloro-1-(2,2, 2–trifluoroethyl)-5-(2-fluorophenyl)-1,3-dihydro-2H-1,4-benzodiazepin-2-one.
5. 7-chloro-1-(2,2, 2–trifluoroethyl)-5-(2-fluorophenyl)-1,3-dihydro-3-hydroxy-2H-1,4-benzodiazepin-2-one.
6. 7-chloro-1-(2,2, 2–trifluoroethyl)-5-(2-fluorophenyl)-1,3-dihydro-2H-1,4-benzodiazepin.
7. 7-chloro-1-(2,2, 2–trifluoroethyl)-5-(2-chlorophenyl)-1,3-dihydro-2H-1,4-benzodiazepin.
8. 7-bromo-1-(2,2, 2–trifluoroethyl)-5-(2-fluorophenyl)-1,3-dihydro-2H-1,4-benzodiazepin.
9. 7-chloro-1-(2,2, 2–trifluoroethyl)-5-(2-fluorophenyl)-1,3-dihydro-3-hydroxy-2H-1,4-benzodiazepin-2-thione.

Of particular interest in relation to the present invention are compound Nos. 3 and 1, and Nos. 4 and 2, which are metabolites of Nos. 3 and 1 respectively.

The trifluorobenzodiazepines referenced above are also lipid soluble. All of the benzodiazepines reported to have $BZ_1$ specificity have a $CH_2CF_3$ group on the nitrogen in the "B" ring. Metabolic loss of this $C_2CF_3$ group results in a benzodiazepine that is non-specific for the $BZ_1$-$BZ_2$ receptors. Applicants' invention was made possible by the unexpected and surprising discovery from pharmacokinetic studies that sublingual dosing minimizes the desalkylation metabolic pathway leading to the formation of nonspecific metabolites of the selected trifluorobenzodiazepine.

An object of the present invention is to increase the effectiveness of certain selected trifluorobenzodiazepines on human subjects to reduce anxiety and convulsions.

Another object of the present invention is to provide a new administration method which increases the

availability of certain selected trifluorobenzodiazepines to the human central nervous system and decreases the amount of undesirable metabolites available thereto.

Still another object of the present invention is to maximize the effect of certain selected trifluorobenzodiazepines on $BZ_1$ receptorsof the human central nervous system and minimize their effect on $BZ_2$ receptors.

Intraoral formulations suitable for use in formulating trifluorobenzodiazepines, for use in the context of the present invention, are of standard type, especially since the speed of dissolution, and therefore of onset of pharmaceutical effect, is not of relevance of the present invention; speed of dissolution, in contrast to location of absorption, is not thought to have any effect on the enhancement of the therapeutic effects of trifluorobenzodiazepines. Suitable formulation techniques are widely described. Examples are Pharmaceutical Sciences, 1985, p. 1604, and mentioned in the U.S. Pharmacopoeia, Volume XIX, p. 650.

As mentioned in the Pharmaceutical Sciences reference, buccal or sublingual (intraoral) tablets are generally small, flat tablets; their shape is adapted for dissolution on the mouth. Such tablets may thus be preferred for use in the present invention. Such tablets may have other adaptations, compared with tablets intended to be swallowed. Thus, it may be particularly desirable that a tablet for intraoral administration be sugar coated and/or contain a palatability-enhancing agent. It may have different dissolution characteristics. It may contain a different, probably lower, dose of active ingredient, in view of enhanced therapeutic effect.

Test results will now be described. Testing was carried out using volunteer adult humans. Standard tablets containing, as active ingredient, solely quazepam or solely halazepam, were used. Such tablets were intended by their manufacturers to be swallowed but dissolved adequately in the mouth to permit the intraoral testing.

Figure 1 is a graph illustrating the concentration of quazepam (Q) and N-desalkyl-2-oxoquazepam (DOQ) in the blood plasma over 96 hours following a single sublingual dose (SL) or per oral swallowed dose (PO) of quazepam in a 15 mg standard tablet.

Figure 2 is a graph illustrating the concentration of quazepam (Q) and N-desalkyl-2-oxoquazepam (DOQ) in the blood plasma over 210 hours following a single sublingual dose (SL) or per oral swallowed dose (PO) of quazepam in a 15 mg standard tablet.

Figure 3 is a graph of computer simulated concentration levels of quazepam and N-desalkyl-2-oxoquazepam in the blood following sublingual and oral swallowed doses of quazepam (15 mg standard tablet) once a day for a 15 day period illustrating the marked reduction in accumulated levels of desalkyloxoquazepam with sublingual dosing;

Figure 4 is a graph illustrating the concentration of halazepam (HZ) and N-desalkyl-3-hydroxy-halazepam (ND) in the blood over 96 hours following a single sublingual dose (SL) or per oral swallowed dose (PO) of halazepam in a 20 mg standard tablet.

Figure 5 is a graph illustrating the concentration of halazepam (HZ) and N-desalkyl-3-hydroxy-halazepam (ND) in the blood over 240 hours following a single sublingual dose (SL) or per oral swallowed (PO) of halazepam in a 20 mg standard tablet; and

Figure 6 is a flow chart of the method of the present invention.

Quazepam, a trifluorobenzodiazepine, is selective for benzodiazepine Type I ($BZ_1$) receptors of the central human nervous system. Action at the $BZ_1$ receptors has been linked to antianxiety and anticonvulsant and/or hypnotic effects, whereas action at $BZ_2$ receptors of the human central nervous system has been linked to muscle relaxation and ataxic effects. N-desalkyl-2-oxoquazepam (DOQ), an active metabolite of quazepam (Q), is $BZ_1$, $BZ_2$ receptor non-specific, and also has a much higher affinity or potency for both receptor types when compared to the $BZ_1$ specific affinity of quazepam (Q). Thus, the higher affinity metabolite (DOQ) of quazepam (Q) contributes substantially to the adverse ataxic and incoordination effects of quazepam on the human central nervous system. In addition, because DOQ has a much longer elimination half-life than the parent quazepam compound (Q), repeated dosing leads to the gradual accumulation of the non-specific, unwanted metabolite, and a greater ratio of DOQ/Q attains over a period of days. Thus, after two to three hours subsequent to an acute dose of quazepam, the DOQ metabolite, both because of its increase gradual accumulation and its greater potency than the parent compound Q, can obviate the advantages of quazepam itself.

Applicants have unexpectedly and surprisingly discovered that sublingual dosing, in contrast to the usual clinical oral dosing of quazepam, increases the availability of quazepam about 60% while the DOQ drops to about one-half that of the oral quazepam administration levels. In other words, applicants have unexpectedly and surprisingly discovered that the aforementioned undesirable "first pass" augmentation of dealkylation to the DOQ metabolite can be markedly reduced or obviated by sublingual dosing of quazepam. This change in concentrations for the two compounds can be seen with reference to Figure 1 and Figure 2 of the drawings where the differences in the parent compound Q and the metabolite DOQ for

both the oral and sublingual dosing is shown. In Figure 3, by use of standard mutliple Q dose simulations, the differences in accumulation of Q and DOQ for sublingual versus oral dosing over 15 days is shown. With chronic dosing it is readily apparent that after 15 days the DOQ level, following oral administration, has reached levels that are associated with the threshold for impairing ataxic and incoordination affects (especially if larger doses are given). With sublingual dosing the accumulated levels of DOQ are approximately one-half of the oral dosing and the levels of Q are over twice that of the oral levels.

In Table 1 and Table 2, set forth below, the average pharmacokinetic parameters for both Q and DOQ for both oral and sublingual routes of administration are set forth:

TABLE I

AVERAGE PHARMACOKINETIC PARAMETERS OF QUAZEPAM FOLLOWING SUBLINGUAL AND ORAL ADMINISTRATION OF QUAZEPAM (15 mg)

| | Route of Administration of Quazepam | |
|---|---|---|
| Parameter | Sublingual | Oral |
| $t_{1/2}$ $K_a$ (hr) | 0.27 ± 0.10[a] | 0.77 ± 0.23 |
| $t_{1/2}$ $\lambda_1$ (hr) | 1.44 ± 0.45 | 1.73 ± 0.65 |
| $t_{1/2}$ $\lambda_2$ (hr) | 27.72 ± 7.18 | 24.63 ± 8.35 |
| Lag time (hr)[b] | 0.18 ± 0.05 | 0.52 ± 0.28 |
| $C_{max}$ (ng/ml)[b] | 42.35 ± 10.43 | 26.74 ± 6.83 |
| $t_{max}$ (hr)[b] | 0.78 ± 0.31 | 2.57 ± 1.69 |
| AUC (ng.hr/ml)[b] | 1461.35 ± 298.67 | 472.79 ± 238.92 |
| CL/F (1/hr)[b] | 8.78 ± 5.25 | 37.56 ± 16.89 |

[a] Mean ± SD
[b] Differed significantly from oral dosing ($P<0.05$)

Legend:
$t_{1/2}$ = Half-Life
$K_a$ = Absorption
$\lambda_1$ = Rapid Distribution
$\lambda_2$ = Terminal Elimination
$C_{max}$ = Peak Plasma Concentration
$t_{max}$ = Time to $C_{max}$
AUC = Area Under Plasma Concentration-Time Curve
CL/F = Clearance

TABLE II

| AVERAGE PHARMACOKINETIC PARAMETERS OF N-DESALKYL-2-OXOQUAZEPAM FOLLOWING SUBLINGUAL AND ORAL ADMINISTRATION OF QUAZEPAM (15 mg) | | |
|---|---|---|
| | Route of Administration of Quazepam | |
| Parameter | Sublingual | Oral |
| $t_{1/2}$ $K_m$ (hr) | 1.07 ± 0.31[a] | 1.24 ± 0.52 |
| $t_{1/2}$ $\lambda_2$ (hr) | 69.30 ± 18.62 | 71.44 ± 21.16 |
| Lag time (hr) | 1.74 ± 0.86 | 0.66 ± 0.32 |
| $C_{max}$ (ng/ml)[b] | 8.18 ± 2.35 | 17.58 ± 4.17 |
| $t_{max}$ (hr) | 7.33 ± 4.15 | 6.17 ± 3.52 |
| AUC (ng.hr/ml)[b] | 949.02 ± 365.74 | 1966.70 ± 410.90 |

[a] Mean ± SD
[b] Differed significantly from oral dosing ($P<0.05$)
Legend:
$t_{1/2}$ = Half-Life
$K_m$ = Formation
$\lambda_2$ = Terminal Elimination
$C_{max}$ = Peak Plasma Concentration
$t_{max}$ = Time to $C_{max}$
AUC = Area Under Plasma Concentration-Time Curve

The profile in Figures 1 and 2 of the drawings clearly shows that there is a first-pass metabolism for Q leading to the attenuated Q levels. On the basis of applicants' pharmacokinetic studies, applicants have discovered that sublingual dosing which bypasses first pass metabolism, minimizes the N-dealkylation metabolic pathway that leads to the formation of the unwanted metabolite, DOQ. This has lead applicants to the sublingual dosing method of the invention which provides for maximization of the important therapeutic effects of the drug. Thus, applicants have discovered the means by which quazepam can be administered such that one can maximize the $BZ_1$ effects and reduce the $BZ_2$ effect of its metabolite (DOQ) and thereby enhance the efficacy in use on humans of this therapeutic drug.

In summary, applicants have discovered the following: (1) The use of sublingual dosing of quazepam to markedly reduce first pass metabolism of the quazepam structure and thereby enhance the $BZ_1$ effect of the drug; and (2) The use of sublingual dosing to increase the $BZ_1$-$BZ_2$ ratio with acute dosing and repeated dosing over days (since the dosing regimen is reducing the DOQ levels and thus attenuating the many impairing effects of the high affinity slowly metabolized quazepam metabolite). These phenomenon resulting from sublingual dosing provide for an unexpected and surprising enhancement of the efficacy and reduction of toxicity of the drug in reducing anxiety and convulsions in humans.

With references now to Figures 4 and 5, applicants have also tested high $BZ_1$ specific drug halazepam and discovered similar results obtained by sublingual administration thereof: The availability of halazepam was significantly increased thus maximizing the $BZ_1$ effect while reducing the $BZ_1$-$BZ_2$ metabolite N-dealkyl-hydroxy-halazepam. Applicants thus believe that intraoral administration, either buccal or sublingual, of selected trifluorobenzodiazepines can substantially enhance their therapeutic effect for the reasons set forth herein. Applicants' novel method can be better appreciated with reference to Figure 6 of the drawings which depicts a flow chart of the steps of the novel therapeutic method.

It will be understood that various details of the invention may be changed without departing from the scope of the invention. Furthermore, the foregoing description is for the purpose of illustration only, and not for the purpose of limitation--the invention being defined by the claims.

It will be understood that the following claims, in referring to intraoral administration, are not intended to cover incidental intraoral administration of a minor quantity of a therapeutic agent, for example before a tablet is swallowed. Equally, it will be understood that when a therapeutic agent is administered intraorally in the present invention, that a minor quantity can be expected to pass to the stomach, even though the

patient retains the tablet in the mouth until dissolution is complete.

Tablets for use in relation to the invention may be packaged with instructions for intraoral administration.

## Claims

1. A 1-N-trifluorobenzodiazepine compound for use in a method of therapy, by intraoral administration thereof to a human patient.
2. A substantially lipid soluble 1-N-trifluoroalkylbenzodiazepine compound, which has one or more unwanted or adverse N-desalkylated metabolites the formation of which is/are enhanced by portal vein entry to the liver, for use in a method of therapy such as to reduce the formation of such metabolite(s), involving intraoral administration thereof to a human patient.
3. A substantially lipid soluble 1-N-trifluorobenzodiazepine compound, which is $BZ_1$ receptor specific and has one or more unwanted or adverse $BZ_1$, $BZ_2$ receptor non-specific metabolites that are increased by portal vein entry to the liver, for use in a method of therapy which substantially bypasses the portal vein entry to the liver, involving intraoral administration to a human patient of a therapeutically effective amount of an intraoral formulation.
4. A substantially lipid soluble 1-N-trifluoroalkylbenzodiazepine compound, which has one or more acceptable metabolites with the trifluoroalkyl moiety and one or more unwanted N-desalkylated metabolites, for use in a method of therapy to obtain maximal desired antianxiety and/or anticonvulsant and/or hypnotic effects and minimal undesired ataxic effects, by administering said compound to a human patient substantially by intraoral administration and substantially not by absorbtion following swallowing.
5. A compound as claimed in any preceding claim, wherein the compound is of general formula

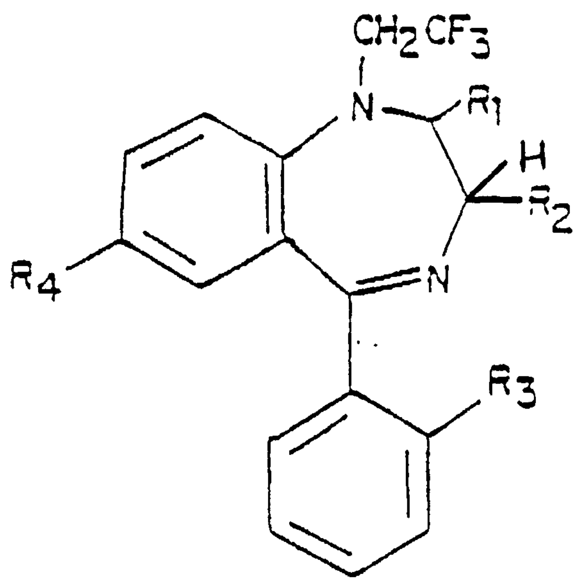

wherein $R^1$ represents the moieties =O or =S or -H, -H;
$R^2$ represents a hydrogen atom or a hydroxy group;
$R^3$ represents a hydrogen, fluorine or chlorine atom;
and
$R^4$ represents a bromine or chlorine atom.

6. A compound as claimed in Claim 5, selected from the group comprising: 7-chloro-1-(2,2,2-trifluoroethyl)-5-phenyl-1,3-dihydro-2H-1,4-benzodiazepine-2-one (halazepam), 7-chloro-1-(2,2,2-trifluoroethyl)-5-phenyl-1,3-dihydro-3-hydroxy-2H-1,3-benzodiazepine-2-one (3-OH-halazepam), 7-chloro-1-(2,2,2-trifluoroethyl)-5-(2-fluorophenyl)-1,3-dihydro-2H-1,4-benzodiazepine-2-thione (quazepam), 7-chloro-1-(2,2,2-trifluoroethyl)-5-(2-fluorophenyl)-1,3-dihydro-2H-1,4-benzodiazepine-2-one (2-OXO-Q), 7-chloro-1-(2,2,2-trifluoroethyl)-5-(2-fluorophenyl)-1,3-dihydro-3-hydroxy-2H-1,4-benzodiazepine (2-OXO-3-OH-Q), 7-chloro-1-(2,2,2-trifluoroethyl)-5-(2-fluorophenyl)-1,3-dihydro-2H-1,4-benzodiazepine (SCH 15698), 7-chloro-1-(2,2,2-trifluoroethyl)-5-(2-chlorophenyl)-1,3-dihydro-2H-1,4-benzodiazepine (SCH 15893), 7-bromo-1-(2,2,2-trifluoroethyl)-5-(2-fluorophenyl)-1,3-dihydro-2H-1,4-benzodiazepine (SCH 18449), 7-chloro-1-(2,2,2-trifluoroethyl)-5-(2-fluorophenyl)-1,3-dihydro-3-hydroxy-2H-1,4-benzodiazepine-2-thione (3-OH-Q).

7. A compound as claimed in Claim 6, which compound is quazepam.

8. A compound as claimed in Claim 6, which compound is halazepam.

9. A composition for intraoral administration to a human, comprising a compound as claimed in any preceding claim, the composition being for use in a method of therapy involving intraoral administration.

10. Use of a compound as claimed in any of Claims 1 to 8, or of a composition as claimed in Claim 9, in the manufacture of a medicament for use in a method of achieving enhanced therapeutic effects by intraoral administration.

Figure 1. Sublingual VS Oral Q and DOQ, Single Dosing

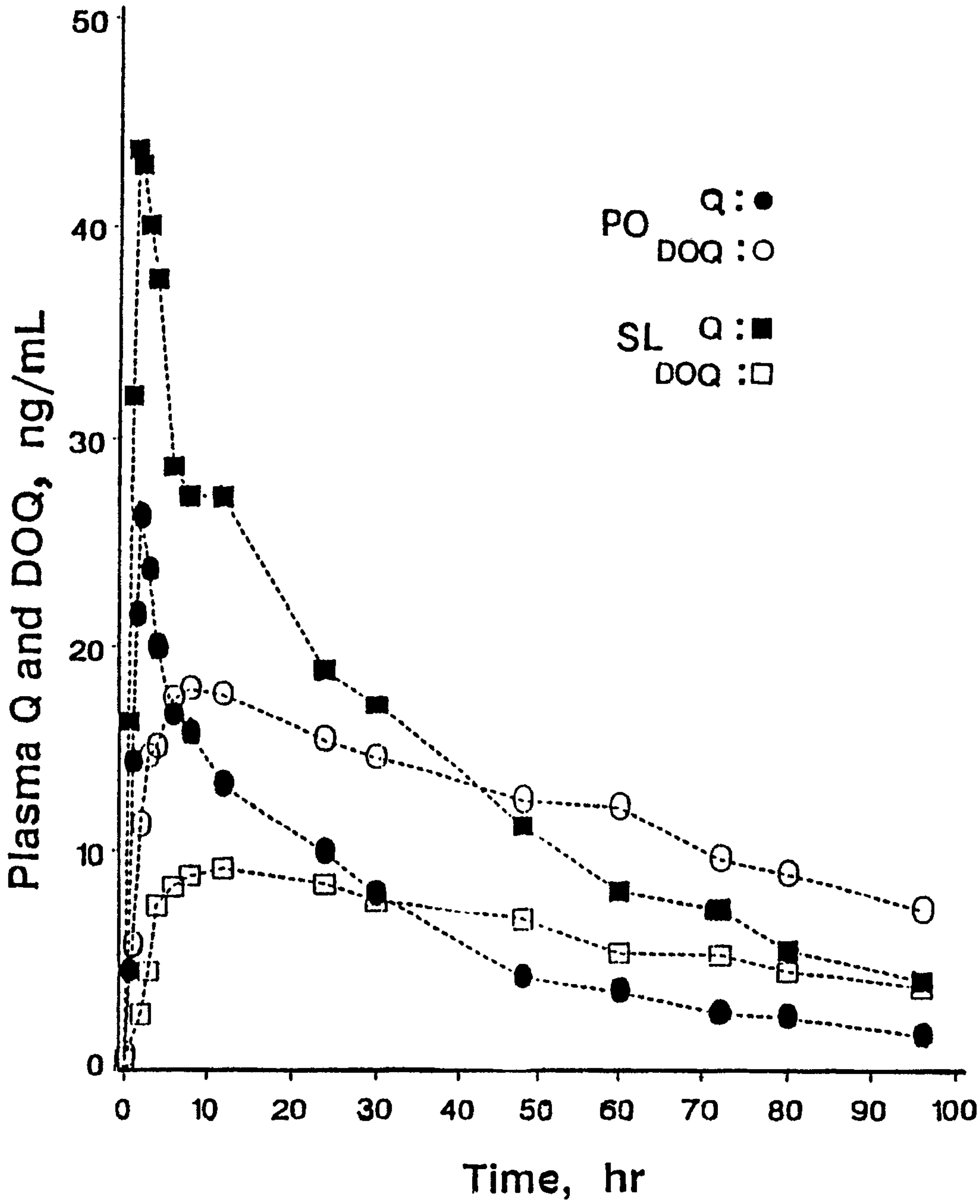

Figure 2. Sublingual VS Oral Q and DOQ, Single Dosing

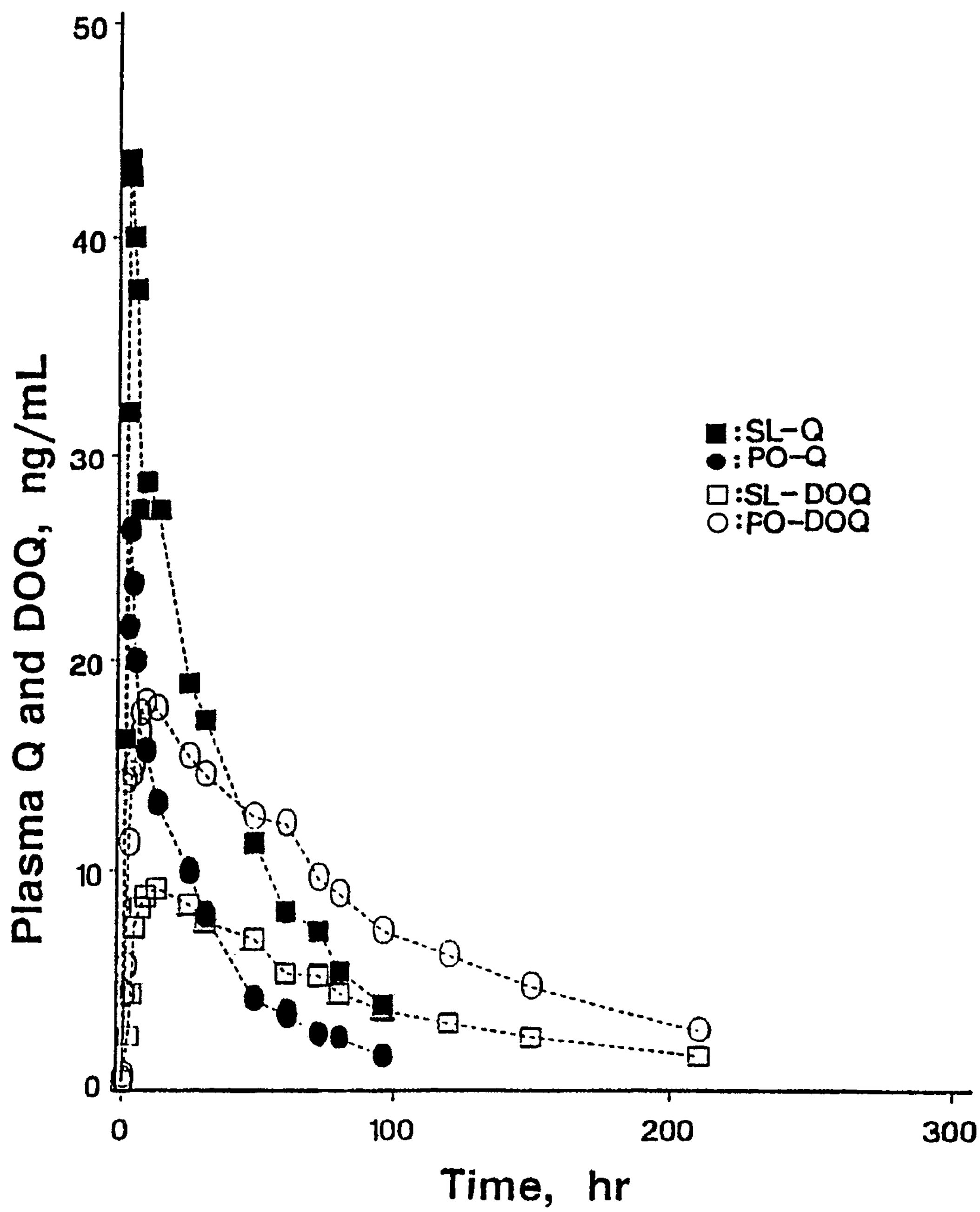

Figure 3.Sublingual VS Oral Q and DOQ,Multiple Dosing

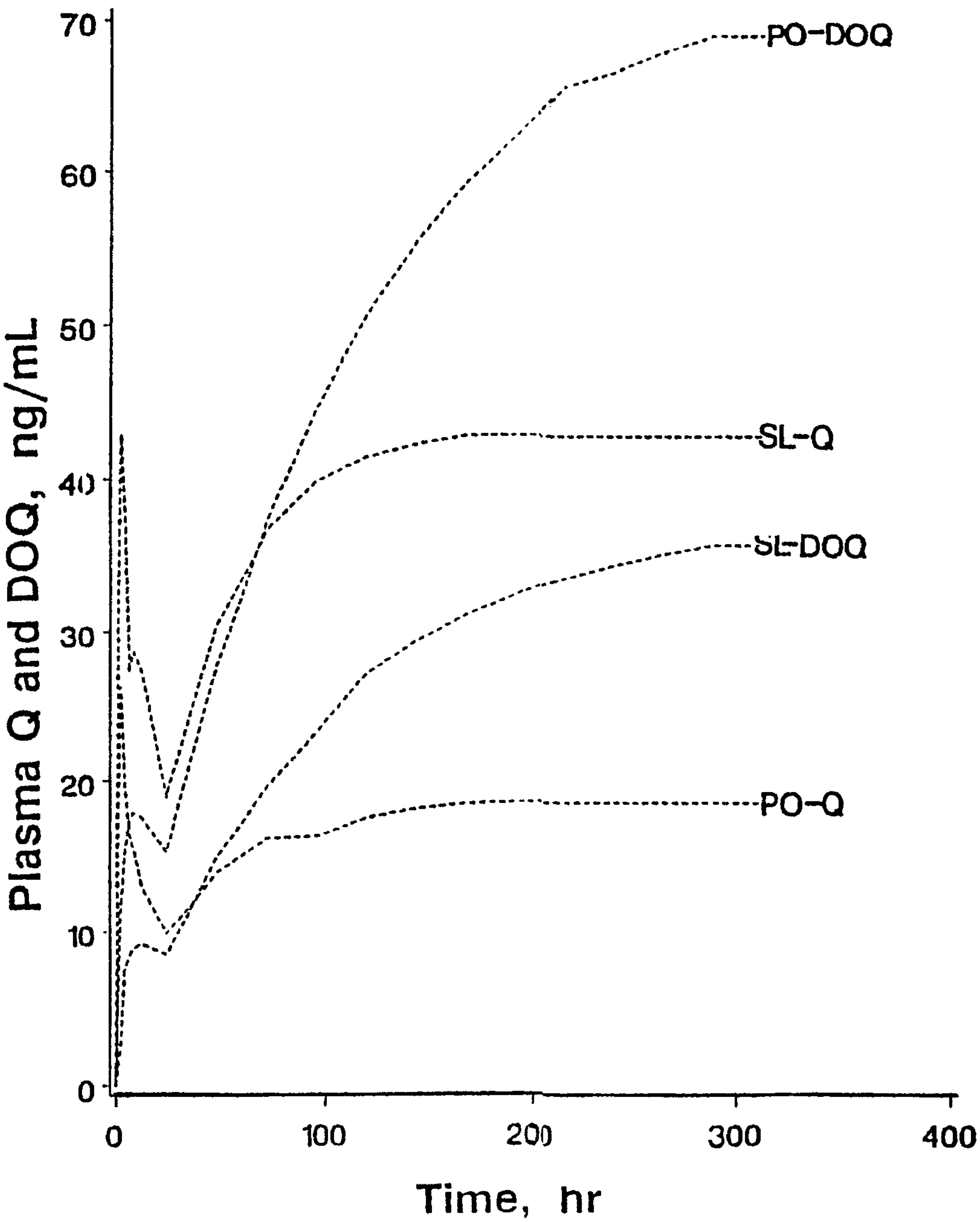

Figure 4.Sublingual VS Oral HZ and NDZ, Single Dosing

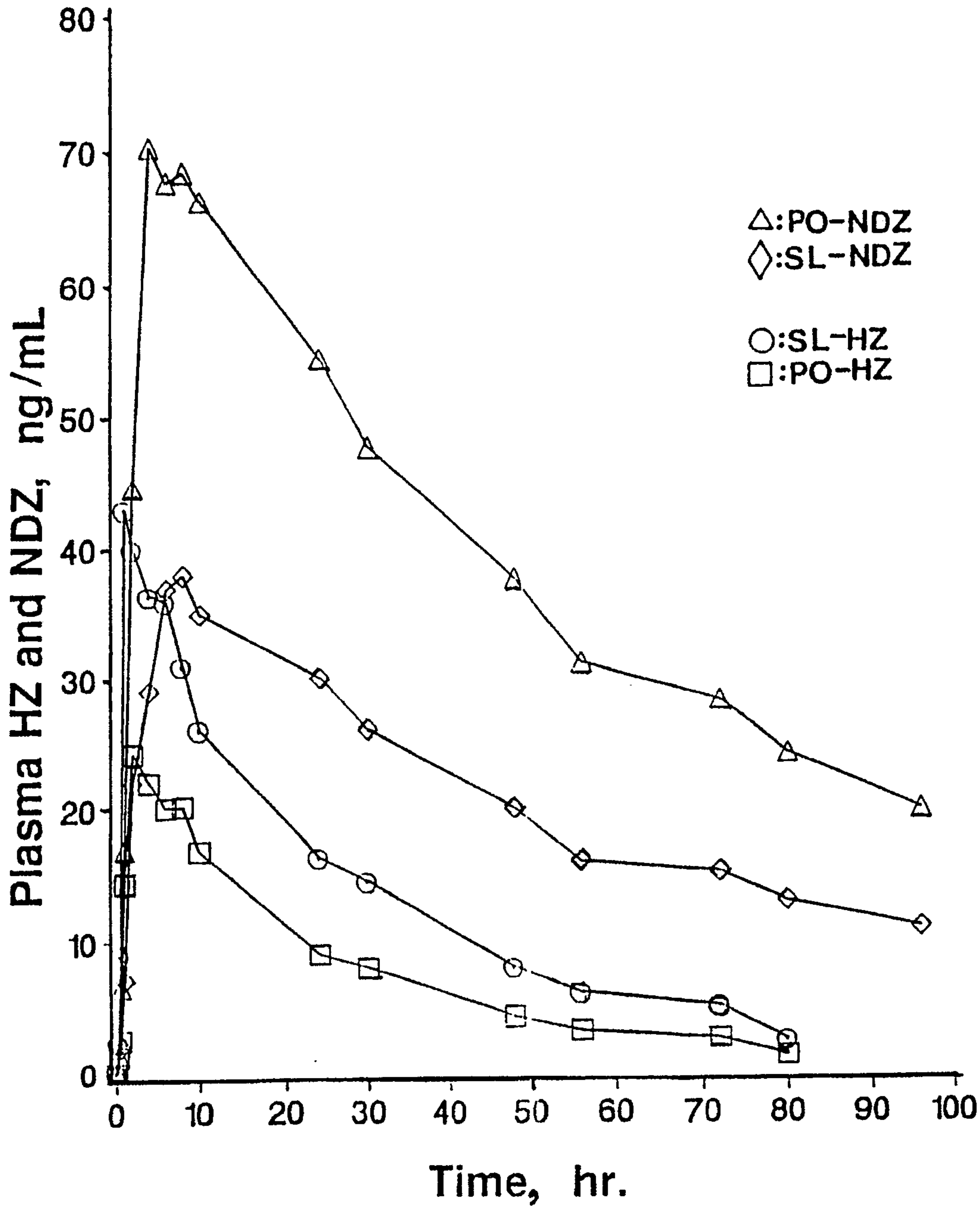

Figure 5.Sublingual VS Oral HZ and NDZ,Single Dosing

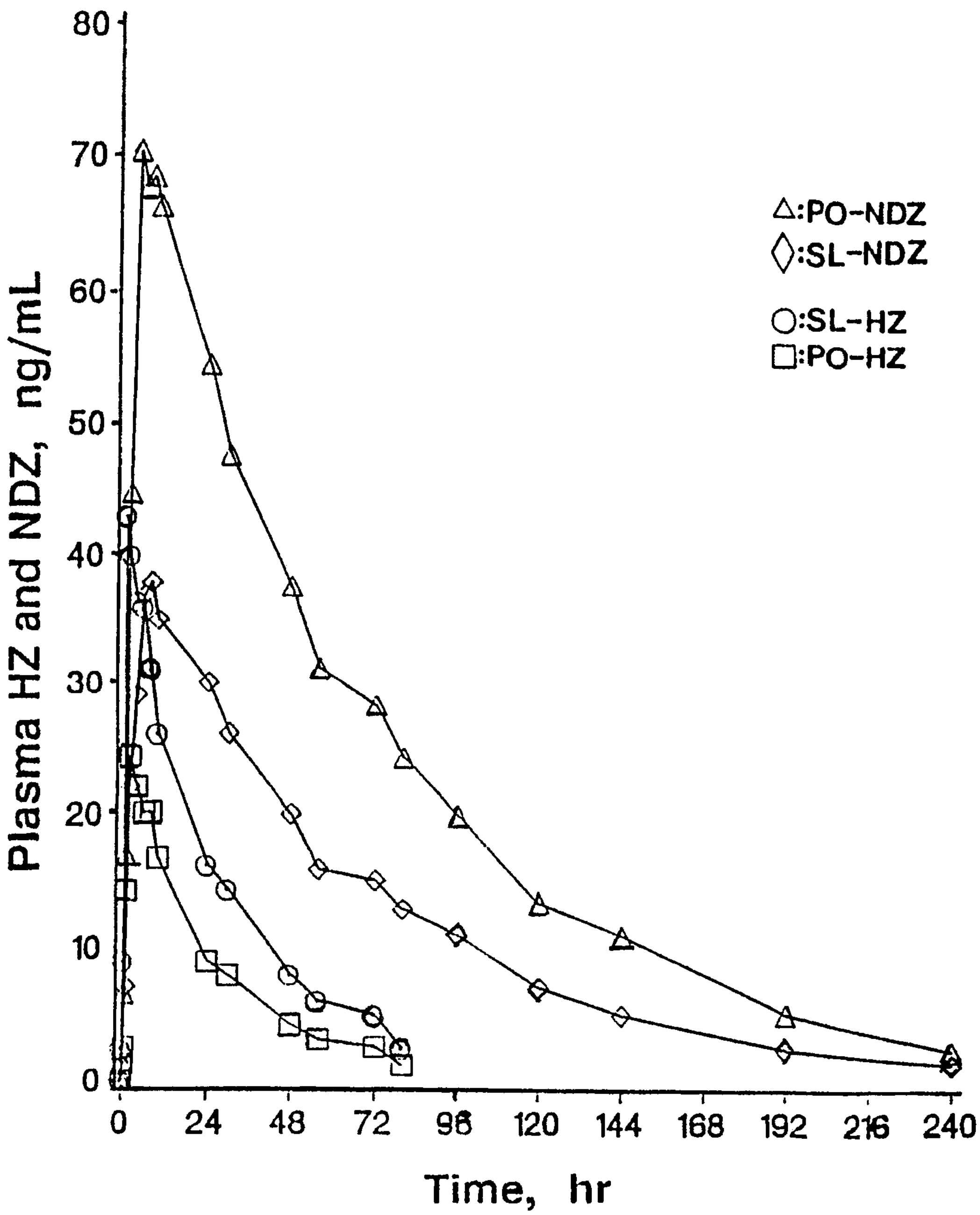

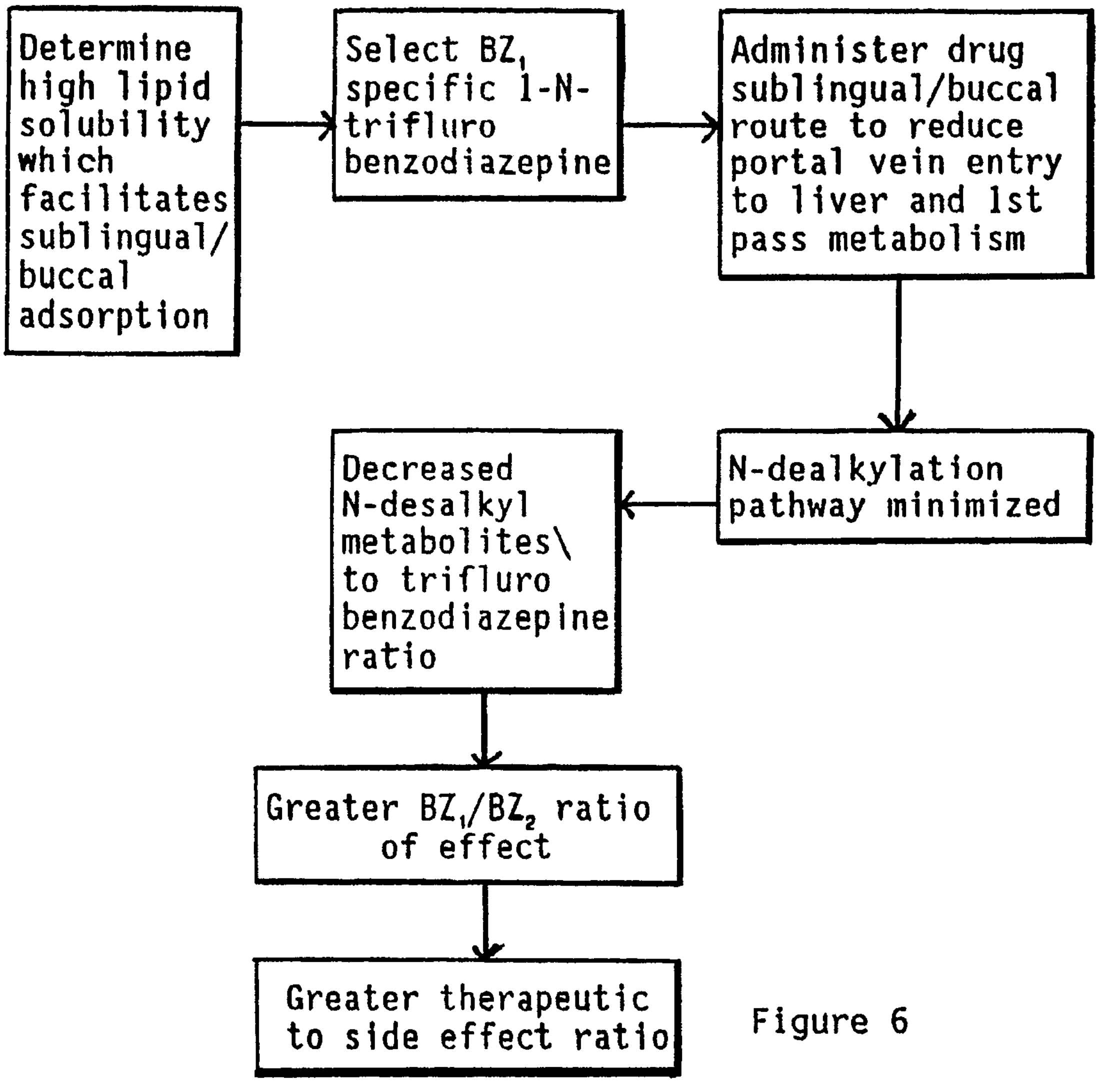
Determine high lipid solubility which facilitates sublingual/ buccal adsorption
Select $BZ_1$ specific 1-N-trifluro benzodiazepine
Administer drug sublingual/buccal route to reduce portal vein entry to liver and 1st pass metabolism
N-dealkylation pathway minimized
Decreased N-desalkyl metabolites\ to trifluro benzodiazepine ratio
Greater $BZ_1/BZ_2$ ratio of effect
Greater therapeutic to side effect ratio

Figure 6